# EUROPÄISCHE PATENTSCHRIFT

(11) **EP 0 355 513 B1**
(45) Veröffentlichungstag und Bekanntmachung des Hinweises auf die Patenterteilung: **21.09.1994**
(21) Anmeldenummer: 89114312.5
(22) Anmeldetag: 03.08.1989
(51) Int. Cl.: C07C 39/16, C07C 37/11

(54) **Isomerisierung oder Homologisierung von Bisphenolen**
Isomerisation or homologisation of bisphenols
Isomérisation ou homologisation de bisphénols

(30) Priorität: 16.08.1988 DE 3827643
(43) Veröffentlichungstag der Anmeldung: 28.02.1990
(73) Patentinhaber: BAYER AG, 51368 Leverkusen (DE)
(72) Erfinder: Rudolph, Udo, Dr., D-4150 Krefeld (DE); Wulff, Claus, Dr., D-4150 Krefeld (DE)

(56) Entgegenhaltungen:
- GB-A- 1 066 137
- GB-A- 1 474 168
- US-A- 4 156 790
- Römpps Chemie-Lexikon, 8. Auflage, 1979, S.454-455
- Dictionary of organic compounds, fourth , completely revised,enlarged and re-set edition in five volumes, 1965, Seite 406

## Beschreibung

Die Erfindung betrifft ein Verfahren zur Herstellung von Bisphenolen und Bisphenolgemischen durch Isomerisierung oder Homologisierung von Bisphenolen an sauren oder alkalischen Katalysatoren in Gegenwart entsprechender Phenole.

Bisphenole sind wichtige Rohstoffe für Chemiewerkstoffe, z.B. für Epoxidharze und Polycarbonate auf der Basis von z.B. Bisphenol A. Zur Modifizierung der Polycarbonateigenschaften können diesen Polymeren für bestimmte Anwendungen andere Polycarbonate, z.B. auf der Basis anderer Bisphenole zugemischt werden, z.B. zur Erhöhung der Flammwidrigkeit Polycarbonate auf der Basis von Tetrachlor- bzw. Tetrabrombisphenol A usw., zur Erhöhung der Wärmeformbeständigkeit Polycarbonate auf der Basis von Tetramethylbisphenol usw.

Diese Verfahren sind sehr umständlich. Sie erfordern neben Polycarbonat auf der Basis von Bisphenol A die zusätzliche Herstellung eines anderen Bisphenols und des entsprechenden Polycarbonats in ausreichender Menge. Es sind neben Bisphenol A nur wenige andere Bisphenole im Markt, so daß in aller Regel eine neue Verfahrensentwicklung erforderlich ist. Zusätzlich erfordert die Mischung von Polymeren z.B. einen zusätzlichen Extrusionsschritt.

Aus der GB-A 1 474 168 ist die Isomerisierung von Bisphenol-A in Gegenwart von Chlorwasserstoff unter erhöhtem Druck bekannt.

Aus der US-A 4 156 790 ist die Isomerisierung von Dihydroxydiphenylchlorethylen in das para, para-Isomere in Gegenwart von Trifluormethansulfonsäure bekannt.

Es wurde nun gefunden, daß Bisphenole in Gegenwart eines bestimmten Phenols, z.B. 2.6-Dimethylphenol, in Gegenwart eines geeigneten Katalysators isomerisieren oder Homologisierung.

Es können z.B. Bisphenol A und Alkylphenole in 3.5-Dimethyl-4.4'-dihydroxydiphenyl-2.2-propan und/oder 3.3', 5.5'-Tetramethyl-4.4-dihydroxydiphenyl-2.2-propan und/oder 1.1.3.4.6-Pentamethyl-3-(3.5-dimethyl-4-hydroxyphenyl)-indan-5-ol überführt werden.

Gegenstand der Erfindung ist daher ein Verfahren zur Isomerisierung oder Homologisierung von Bisphenolen der Formel IV 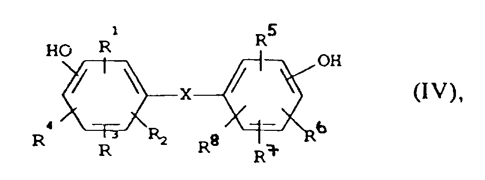
in welcher
- R¹-R⁸: unabhängig voneinander gleich oder verschieden sind und für Wasserstoff, ein bis vierfach C₁-C₁₂-Alkyl stehen, X für einen C₁-C₁₂-Alkyliden- und C₃-C₁₂-Cycloalkylidenrest ortho- oder paraständig zur OH-Gruppe steht,
in Gegenwart von Phenolen der Formel V 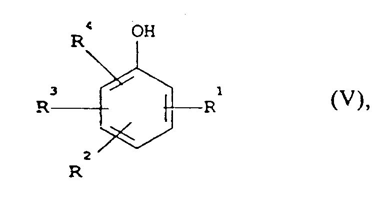
in welcher
R¹, R², R³ und R⁴ unabhängig voneinander für Wasserstoff, C₁-C₁₂-Alkyl, Halogen, C₆-C₂₄-Aryl, C₁-C₁₂-Alkoxy, C₅-C₃₀-Cycloalky steht, dadurch gekennzeichnet, daß unter Normaldruck mit β-Mercaptopropionsäure, Mercaptoaminen oder Thiazolidinen belegte Ionenaustauscher-harze als Katalysatoren eingesetzt werden.

So kann erfindungsgemäß z.B. Bisphenol A leicht in Gegenwart von 2,6-Dimethylphenol in Gegenwart eines geeigneten Katalysators in folgende Bisphenole isomerisiert werden:
3.5-Dimethyl-4.4'-dihydroxydiphenyl-2.2-propan (I) 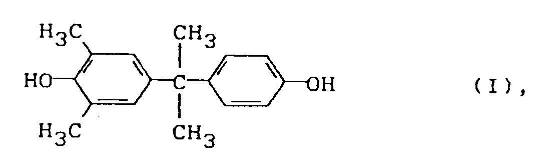
3.3', 5.5'-Tetramethyl-4.4'-dihydroxydiphenyl-2.2-propan (II) 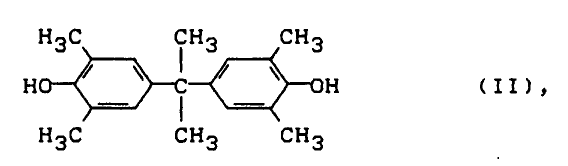
1.1.3.4.6-Pentamethyl-3-(3.5-dimethyl-4-hydroxyphenyl)-indan-5-ol (III)

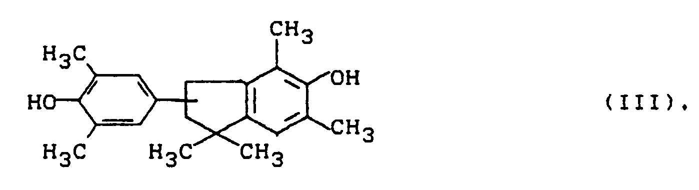

Z.B. aus Bisphenol A erhält man an geeigneten Katalysatoren in Gegenwart anderer Phenole andere Bisphenole entsprechend. Geeignete andere Phenole weisen mindestens eine nicht weiter substituierte Stellung am aromatischen Ring auf. Beispielsweise seien genannt; Mono- oder ein bis vierfach C₁-C₁₂-alkylsubstituierte Phenole wie o-, m- und p-Methylphenol, o-, m- und p-Ethylphenol usw., 2.6-Dimethylphenol usw., Mono- oder mehrfach halogenierte Phenole (z.B. mit Cl, Br) wie o-, m- und p-Chlorphenol, o-, m- und p-Bromphenol, 2.6-Dichlorphenol, 2.6-Dibromphenol usw., Mono- oder mehrfach C₁-C₁₀-alkoxysubstituierte Phenole, mono-oder mehrfach C₆-C₂₄-aryl-bzw. C₅-C₃₀-cycloalkylsubstituierte Phenole usw.. Mehrkernphenole sind ebenfalls erfindungsgemäß einsetzbar.

Erfindungsgemäß können Bisphenole der Formel (IV) 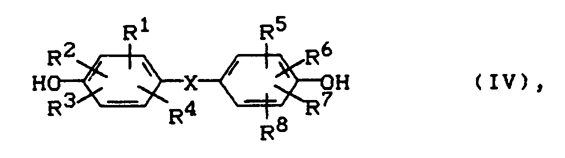
in welcher
- R¹-R⁸: unabhängig voneinander gleich oder verschieden sind und für Wasserstoff, C₁-C₁₂-Alkyl stehen, X für einen C₁-C₁₂-Alkyliden- und C₃-C₁₂-Cycloalkylidenrest ortho- oder paraständig zur OH-Gruppe steht,
eingesetzt werden.

Als Beispiele seien für Bisphenole der Formel (IV) genannt 2.2-Bis-(4-hydroxyphenol)-propan (Bisphenol A), 2.2-Bis-(4-hydroxyphenyl)-butan, 2.4-Bis-(4-hydroxyphenyl)-2-Methylbutan, 1.1-Bis-(4-hydroxyphenyl)-cyclolexan usw., bevorzugt Bisphenol A.

Als Phenole können solche der Formel V 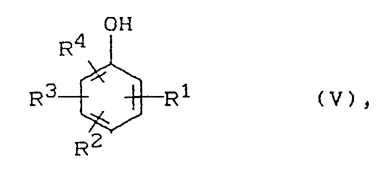
in welcher
- R¹, R², R³ und R⁴: unabhängig voneinander für Wasserstoff, C₁-C₁₂-Alkyl, Halogen (z.B. Cl, Br), C₆-C₂₄-Aryl, C₁-C₁₂-Alkoxy, C₅-C₃₀-Cycloalkyl, bevorzugt C₁-C₁₂-Alkyl
steht,
eingesetzt werden.

Erfindungsgemäße Katalysatoren sind Säuren und Basen, bevorzugt Säuren, beispielsweise Protonensäuren wie Halogenwasserstoffe, z.B. Chlorwasserstoffsäure, Schwefelsäure, Perchlorsäure, Benzolsulfonsäure, Lewis-Säuren wie Bortrifluorid, saure Ionenaustauscherharze u.s.w. Bevorzugt sind saure Ionenaustauscherharze.

Die Katalysatoren können z. B. unter Zusatz von S-haltigen Verbindungen, wie sie z.B. zur Herstellung von Bisphenol A aus Phenol und Aceton bekannt sind, eingesetzt werden. So ist z.B. aus der US-PS 2 468 982, 2 623 908 die Verwendung von z.B. Thioglykolsäure und 3-Mercaptopropionsäure, aus der US-PS 2 359 242 der Zusatz von Thiophenolen, aus der US-PS 2 775 620 der Zusatz von Alkylmercaptanen, aus Chemical Abstracts 58, 1403e der Zusatz von Schwefelwasserstoff bekannt. Die Ionenaustauscherharze können teilweise oder vollständig mit Mercaptoaminen oder Thiazolinen neutralisiert sein (z.B. US-PS 3 394 089).

Geeignete saure Ionenaustauscher sind z.B. handelsübliche Umsetzungsprodukte von Styrol-Divinylbenzol-Copolymerisaten mit üblichen Sulfonierungsmitteln wie Schwefelsäure, Chlorsulfonsäure u.a.. Sie können z.B. in Kugelform vorliegen (Korngrößen von 0,3 bis 1,5 mm Durchmesser). Sie können hinsichtlich des Durchmessers einer Normalverteilung entsprechen oder monodispers sein. Ihre Totalkapazität an Säurefunktionen in wasserfeuchter Form mit einem Wassergehalt von ca. 75 bis 85 Gew.-% reicht von 0,7 bis 2,1 mval/ml Ionenaustauscher, vorzugsweise von 3,5 bis 5 mval, bezogen auf 1 g Trockensubstanz an Ionenaustauscher.

Die erfindungsgemäße Umsetzung wird bei den für die Herstellung von Bisphenol üblichen Temperaturen ausgeführt. So haben sich Temperaturen von 20 bis 150°C bevorzugt 40 bis 80°C bewährt.

Die wasserfeuchten Ionenaustauscher werden vor ihrer erfindungsgemäßen Verwendung gegebenenfalls durch Wärme (20-150°C), gegebenenfalls im Vakuum (bis 10⁻⁵ bar) oder gegebenenfalls durch Waschen mit hydrophilen organischen Flüssigkeiten wie Alkoholen (z.B. Methanol, Ethanol, Propanole) oder Phenolen, oder durch azeotrope Destillation mit organischen Flüssigkeiten wie Toluol, Xylol, Methylenchlorid u.a. getrocknet.

Danach wird das Ionenaustauscherharz mit dem für die Bisphenolherstellung erforderlichen Phenol gespült und in diesem Medium bei Temperaturen oberhalb des Schmelzpunktes dieses Phenols das umzuwandelnde Bisphenol gelöst. Das nach der Umsetzung von Bisphenol und Phenol vorhandene Reaktionsgemisch kann nach üblichen Methoden wie Destillation, Kristallisation usw. aufgearbeitet werden.

Das Verfahren kann diskontinuierlich oder kontinuierlich durchgeführt werden.

Das nach diesem Verfahren hergestellte Bisphenol bzw. Bisphenolgemisch kann für bekannte Anwendungsgebiete eingesetzt werden und eignet sich besonders zur Herstellung von Polycarbonaten und daraus herstellbaren Polymerlegierungen.

### Beispiel 1

228 g Bisphenol A (1 Mol.), 1464 g 2.6-Dimethylphenol (12 Mol.), ca. 250 g dimethylphenolfeuchtes Ionenaustauscherharz Lewatit (Hersteller: Bayer AG) und 0,68 g β-Mercaptopropionsäure werden bei ca. 60°C in einer üblichen Laborapparatur gerührt und der Umsatz gaschromatographisch verfolgt. Nach 6 Stunden haben sich aus dem Bisphenol A zu ca. 25 % 3.5-Dimethyl-4.4'-dihydroxydiphenyl-2.2-propan, zu ca. 10 % 3.3', 5.5'-Tetramethyl-4.4'-dihydroxydiphenyl-2.2-propan und zu ca. 5 % 1.1.3.4.6-Pentamethyl-3-(3.5-dimethyl-4-hydroxyphenyl)-indan-5-ol gebildet. Nach Abfiltrieren des Ionenaustauschers und Abdestillieren des Phenols erhält man weiße Kristalle des oben beschriebenen Bisphenolgemisches.

### Beispiel 2

228 g Bisphenol A (1 Mol.), 1296 g o-Kresol (12 Mol.), ca. 250 g o-kresolfeuchtes Ionenaustauscherharz Lewatit SC 102® (Hersteller: Bayer AG) und 0,68 g β-Mercaptopropionsäure werden bei ca. 60°C in einer üblichen Laborapparatur gerührt und der Umsatz gaschromatographisch verfolgt. Nach 5 Stunden haben sich aus dem Bisphenol A zu ca. 25 % 3-Methyl-4,4'-dihydroxydiphenyl-2.2-propan, zu ca. 20 % 3.3'-Dimethyl-4.4'-dihydroxydiphenyl-2.2-propan und zu ca. 5 % Indane gebildet. Nach Abfiltrieren des Ionenaustauschers und Abdestillieren des Phenols erhält man weiße Kristalle des oben beschriebenen Bisphenolgemisches.

## Patentansprüche

1. Verfahren zur Isomerisierung oder Homologisierung von Bisphenolen der Formel IV 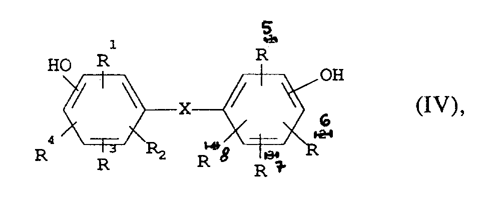 in welcher
R¹-R⁸ unabhängig voneinander gleich oder verschieden sind und für Wasserstoff, ein bis vierfach C₁-C₁₂-Alkyl stehen, X für einen C₁-C₁₂-Alkyliden- und C₃-C₁₂-Cycloalkylidenrest ortho- oder paraständig zur OH-Gruppe steht,
in Gegenwart von Phenolen der Formel V 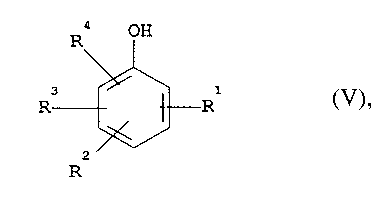 in welcher
R¹, R², R³ und R⁴ unabhängig voneinander für Wasserstoff, C₁-C₁₂-Alkyl, Halogen, C₆-C₂₄-Aryl, C₁-C₁₂-Alkoxy, C₅-C₃₀-Cycloalkyl steht, dadurch gekennzeichnet, daß unter Normaldruck mit β-Mercaptopropionsäure, Mercaptoaminen oder Thiazolidinen belegte Ionenaustauscher-harze als Katalysatoren eingesetzt werden.

## Claims

1. A process for the isomerization or homologization of bisphenols corresponding to formula IV: 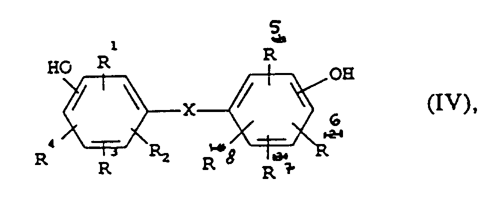 in which
R¹ to R⁸ independently of one another may be the same or different and represent hydrogen or one to four C₁₋₁₂ alkyl groups, X is a C₁₋₁₂ alkylidene or C₃₋₁₂ cycloalkylidene group in the ortho position or para position to the OH group, in the presence of phenols corresponding to formula V: 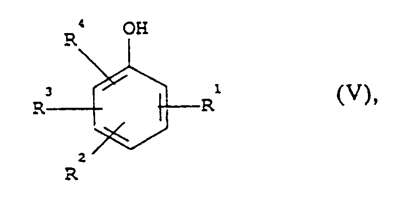 in which
R¹, R², R³ and R⁴ independently of one another represent hydrogen, C₁₋₁₂ alkyl, halogen, C₆₋₂₄ aryl, C₁₋₁₂ alkoxy, C₅₋₃₀ cycloalkyl,
characterized in that ion exchanger resins covered with β-mercaptopropionic acid, mercaptoamines or thiazolidines are used under normal pressure as catalysts.

## Revendications

1. Procédé pour l'isomérisation ou la formation d'homologues de bisphénols de formule IV 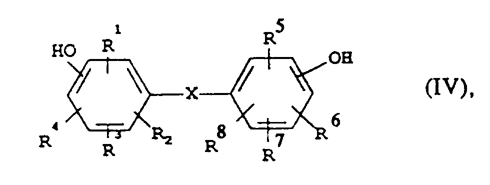 dans laquelle
R¹ à R⁸, ayant des significations identiques ou différentes, représentent chacun, indépendamment les uns des autres, l'hydrogène, un à quatre groupes alkyle en C₁-C₁₂, X représente un coupe alkylidène en C₁-C₁₂ ou cycloalkylidène en C₃-C₁₂ en position ortho ou para du groupe OH,
en présence de phénols de formule V 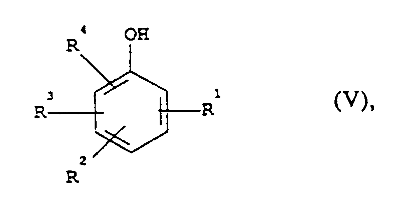 dans laquelle
R¹, R², R³ et R⁴ représentent chacun, indépendamment les uns des autres, l'hydrogène, un groupe alkyle en C₁-C₁₂, un halogène, un groupe aryle en C₆-C₂₄, alcoxy en C₁-C₁₂, cycloalyle en C₅-C₃₀, caractérisé en ce que l'on utilise en tant que catalyseurs des résines échangeuses d'ions chargées d'acide β-mercaptopropionique, de mercaptoamines ou de thiazolidines à pression normale.
